# FASCICULE DE BREVET EUROPEEN

(11) **EP 2 616 013 B1**
(45) Date de publication et mention de la délivrance du brevet: **17.08.2016**
(21) Numéro de dépôt: 11773089.5
(22) Date de dépôt: 15.09.2011
(51) Int. Cl.: A61F 2/40, A61F 2/30, A61F 2/00

(54) **VIS-CAGE POUR LA FIXATION D'UNE CUPULE DE PROTHESE D'EPAULE**
SCHRAUBEN-CAGE ZUR FIXIERUNG EINER PFANNE EINER SCHULTERPROTHESE
SCREW-CAGE FOR FIXING A CUP OF A SHOULDER PROSTHESIS

(30) Priorité: 17.09.2010 FR 1057437
(43) Date de publication de la demande: 24.07.2013
(73) Titulaire: 3S Ortho, 69009 Lyon (FR)
(72) Inventeur: Marc DUPORT, 31400 Toulouse (FR)
(74) Mandataire: Jeannet, Olivier
(86) Numéro de dépôt international: PCT/FR2011/052115
(87) Numéro de publication internationale: WO 2012/035263

(56) Documents cités:
- EP-A1- 1 977 720
- EP-A2- 0 538 895
- EP-A2- 1 900 344
- WO-A1-03/002038
- FR-A1- 2 928 827
- GB-A- 2 199 626
- US-A1- 2004 153 161
- US-A1- 2006 009 852
- US-A1- 2010 191 340

## Description

La présente invention se rapporte à un dispositif pour la fixation d'une prothèse.

En France, environ 10 000 prothèses d'épaule sont posées par an, incluant les prothèses totales d'épaules, dites standards, les prothèses inversées, et plus récemment les prothèses de resurfaçage.

Ce chiffre, en nette augmentation ces dernières années, est en particulier dû à l'augmentation du nombre de pathologies pour lesquelles la pose d'une prothèse d'épaule est maintenant indiquée.

Dans ce contexte, la chirurgie prothétique de l'épaule reste une chirurgie très spécialisée, de haute technicité, nécessitant un apprentissage long et l'utilisation d'ancillaires souvent complexes. Une simplification de ces ancillaires est donc souhaitable afin de faciliter le geste opératoire, de le rendre plus reproductible et donc de diminuer les complications postopératoires. Une telle simplification de l'ancillaire et du geste opératoire aurait également l'avantage de diminuer les coûts de ces opérations de pose de prothèses d'épaule.

Il existe plusieurs types de prothèses d'épaule. Certaines prothèses classiques telles que celle décrite dans US 2004/153161 sont fixées sur l'humérus, grâce à une tige humérale, après une résection conséquente de l'os. L'implantation d'une tige humérale nécessite un évidement important dans l'os pour l'accueillir, diminuant ainsi le capital osseux et fragilisant l'os. La pose d'une telle prothèse est longue et complexe. Après installation de la tige dans l'évidement, l'espace entre l'os et la tige humérale est généralement comblé avec du ciment. Avec le temps, le ciment peut se disloquer et ne plus assurer alors une fixation correcte de la tige.

Il existe également des prothèses de resurfaçage, telles que celle décrite dans FR 2 928 827, qui sont posées sur l'épiphyse de l'humérus après un éventuel ponçage léger de la surface de l'épiphyse. De telles prothèses ne sont pas nécessairement ancrées dans l'os comme décrit précédemment.

Lorsqu'un simple resurfaçage de l'épiphyse n'est pas indiqué, par exemple en cas de fragilité osseuse trop importante, le chirurgien peut choisir de mettre en oeuvre une technique intermédiaire dite de « demi-resurfaçage » ou une partie de l'épiphyse est réséquée. Le chirurgien est alors amené à utiliser une prothèse telle que celle décrite dans le document EP 0538 895, comportant une cupule destinée à remplacer la partie de l'épiphyse réséquée et une vis d'ancrage destinée à être fixée dans l'os par vissage, une telle vis d'ancrage comportant à cet effet un filetage sur toute sa longueur. La cupule est alors fixée sur la vis d'ancrage.

Le positionnement de la vis d'ancrage conditionne le positionnement de la cupule et doit être aussi précis que possible. Il arrive que le patient éprouve un inconfort ou des douleurs parce que la prothèse a été mal positionnée, ce qui oblige alors le chirurgien à réopérer pour repositionner correctement la prothèse. Le retrait de la vis d'ancrage, qui est conçue pour assurer un ancrage solide de la prothèse dans l'os, peut alors occasionner des problèmes et fragiliser l'os.

Le document GB 2 199 626 décrit un implant dentaire de forme générale tubulaire comportant une zone inférieure ajourée, une partie centrale filetée et une partie supérieure lisse formant une tête.

Le document US 2010/191340 décrit une tête prothétique pour humérus comportant une cupule destinée à être équipée d'une vis tubulaire comportant un filetage s'étendant sur toute sa longueur.

Ce document divulgue les caractéristiques du préambule de la revendication 1.

La présente invention a pour objet de remédier en tout ou partie aux différents inconvénients cités précédemment.

Dans ce contexte, la présente invention a pour objet de proposer un dispositif de fixation d'une cupule de prothèse d'épaule qui préserve le capital osseux du patient,
assure une fixation améliorée, précise et rapide de la cupule tout en pouvant être enlevé sans fragiliser l'os.

A cet effet, la présente invention se rapporte à un dispositif pour la fixation d'une cupule de prothèse d'épaule selon la revendication 1.

Ainsi, un dispositif selon l'invention permet de fixer une cupule, standard ou inversée, en cas de fragilité osseuse et lorsqu'il n'est pas possible d'effectuer un simple resurfaçage. Le corps tubulaire du dispositif, lorsqu'il est fixé dans l'os, assure l'ancrage correct du dispositif grâce aux moyens de vissage et la cupule est montée directement ou indirectement sur le dispositif grâce à la coopération des portions de montage complémentaires. La procédure de pose est simplifiée, en particulier puisque l'accouplement de la cupule sur le dispositif est simple. La fixation du dispositif s'effectue de la façon simple suivante : un orifice de longueur réduite, correspondant à la longueur entre l'extrémité distale et la zone intermédiaire est effectué dans l'os puis la partie de la surface périphérique extérieure dépourvue de moyens de vissage (c'est-à-dire la partie de surface périphérique extérieure située entre l'extrémité distale et la zone intermédiaire) est insérée dans l'orifice, l'insertion complète du dispositif dans l'os est ensuite obtenue grâce au moyens de vissage. Lors du vissage, une partie de l'os est insérée dans le corps tubulaire et permet donc de conserver le capital osseux. Les moyens de vissage assurent une fixation adéquate du dispositif dans l'os, tandis que la partie de la surface périphérique extérieure dépourvue de moyens de vissage, participe à l'alignement et le maintien du dispositif dans l'os. En outre, lors du vissage du dispositif, la variation du pas de vis crée un compactage de l'os et améliore la fixation du dispositif.

En cas de besoin, le dispositif peut être enlevé à l'aide d'une scie cloche dont le diamètre intérieur est adapté pour englober les moyens de vissage. Avec la scie cloche la partie de l'os coopérant avec les moyens de vissage est découpée. Etant donné que les moyens de vissage ne s'étendent pas sur la totalité de la longueur du dispositif mais seulement sur une partie, la longueur de la découpe à effectuer avec la scie cloche est réduite par rapport à une vis d'ancrage classique. Ainsi, le capital osseux est préservé et les risques de fragilisation de l'os sont réduits en cas de retrait. Les moyens de vissage facilitent donc l'extraction du dispositif en cas de changement prothétique.

Selon une forme d'exécution, la portion de montage femelle présente une forme tronconique femelle. Une forme tronconique est simple à réaliser et permet d'effectuer un assemblage rapide tout en permettant un alignement correct de la cupule avec le dispositif d'ancrage.

Selon une possibilité, la hauteur du filet du filetage augmente de l'extrémité proximale vers la zone intermédiaire. La surface de coopération entre l'os et le filetage augmente avec la hauteur du filet et améliore ainsi la fixation du dispositif dans l'os.

Selon une forme d'exécution, la zone intermédiaire est située à une distance de l'extrémité proximale égale à une distance comprise entre 20 et 70% de la distance entre les extrémités proximale et distale, de préférence comprise entre 40 et 50%.

Selon une forme d'exécution, le corps tubulaire présente une surface distale reliant les surfaces périphériques intérieure et extérieure et formant un chanfrein. Le chanfrein permet de faciliter la pénétration du dispositif dans l'os lors de son vissage.

Selon une possibilité, la partie de la surface périphérique extérieure s'étendant de la zone intermédiaire jusqu'à l'extrémité distale est sensiblement lisse. Ainsi, une telle surface lisse permet de faciliter le retrait du dispositif sans fragiliser l'os.

Selon une caractéristique de l'invention, la partie de la surface périphérique extérieure s'étendant de la zone intermédiaire jusqu'à l'extrémité distale présente au moins un orifice traversant le corps tubulaire. Un tel orifice permet une meilleure ré-habitation de l'os lors de sa repousse pour solidariser la partie de l'os en contact avec la surface périphérique intérieure avec la partie de l'os en contact avec la surface périphérique extérieure, en particulier sans compromettre l'extraction éventuelle.

L'invention concerne également une prothèse comprenant un dispositif selon l'invention et une cupule comportant une portion de montage mâle conçue pour coopérer directement ou indirectement avec la portion de montage femelle du dispositif.

Selon une forme d'exécution, la prothèse comporte en outre un organe d'accouplement destinée à être disposée entre le dispositif de l'invention et la cupule, l'organe d'accouplement comportant une portion de montage intermédiaire mâle conçue pour coopérer par complémentarité de forme avec la portion de montage femelle du dispositif et une portion de montage intermédiaire femelle conçue pour coopérer par complémentarité de forme avec la portion de montage mâle de la cupule.

L'invention sera bien comprise à l'aide de la description détaillée qui est exposée ci-dessous en regard du dessin annexé, représentant à titre d'exemple non limitatif une forme d'exécution du dispositif et dans lequel :
la figure 1 est une vue schématique en perspective des différents éléments de la prothèse ;
la figure 2 est une vue schématique en coupe partielle et en perspective de la prothèse de la figure 1 ;
la figure 3 est une vue schématique en perspective d'une prothèse de la figure 1 implanté dans une épiphyse humérale.

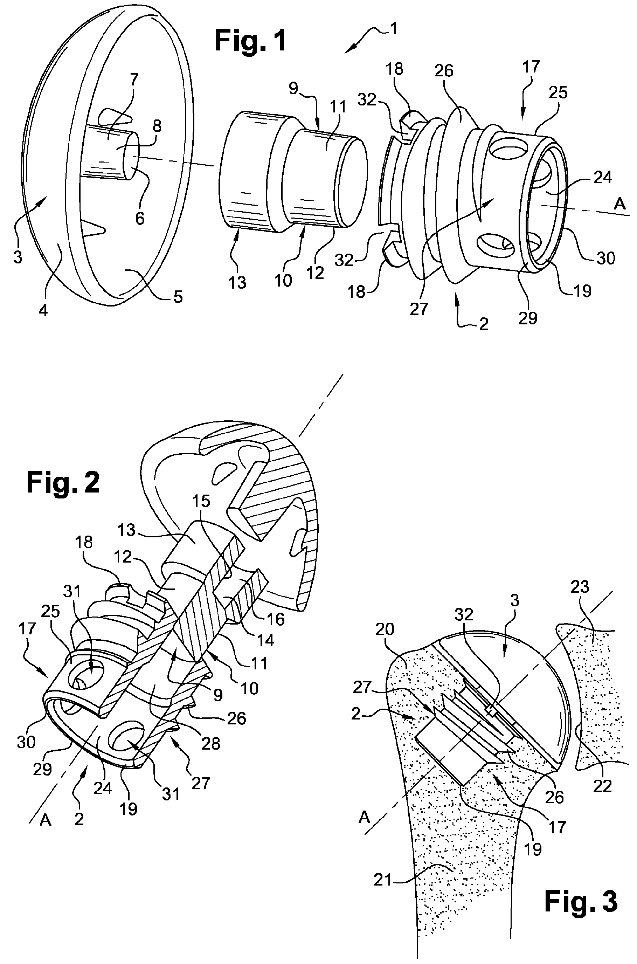

Une prothèse 1, illustrée à la figure 1, comporte un dispositif 2 et une cupule 3 destinée à être montée dans le dispositif 2.

La cupule 3 présente une calotte sphérique 4 creuse, présentant une face intérieure 5 et un plot central 6 tronconique faisant saillie du centre de la face intérieure 5 de la calotte sphérique 4. Le plot central 6 délimite une portion de montage mâle 7 formant un cône morse mâle 8.

Eventuellement, comme c'est le cas de la forme d'exécution illustrée aux figures, la prothèse 1 comprend également un organe d'accouplement 9. Cet organe d'accouplement 9 comporte une première portion 10 de forme sensiblement tronconique présentant une portion de montage intermédiaire mâle 11 formant un cône morse mâle 12. L'organe d'accouplement 9 comporte également une deuxième portion tubulaire 13 munie d'une cavité 14, illustrée à la figure 2, sensiblement tronconique délimitant une portion de montage intermédiaire femelle 15. La portion de montage intermédiaire femelle 15 forme un cône morse femelle 16 dimensionné pour coopérer par complémentarité de forme avec la portion de montage mâle 7 de la cupule 3. Selon la forme d'exécution représentée sur les figures, l'organe d'accouplement 9 présente un cône morse mâle 12 et un cône morse femelle 16 dont les axes de symétrie respectifs sont confondus. De façon alternative et non représentée, le cône morse mâle 12 et le cône morse femelle 16 peuvent présenter des axes de symétries respectifs distincts décalés et/ou non parallèles.

Le dispositif 2 comporte un corps tubulaire 17 s'étendant selon un axe longitudinal A entre une extrémité proximale 18, destinée à coopérer, directement ou indirectement, avec la cupule 3 ou l'organe d'accouplement 9, et une extrémité distale 19 opposée, conçue pour être fixée dans une épiphyse 20 d'un os 21, illustré à la figure 3. Cet os 21 correspond à un humérus lorsque la cupule 3 est conçue pour être disposée dans une cavité glénoïdale 22 d'une scapula 23 partiellement illustrée à la figure 3.

Le corps tubulaire 17 délimite une surface périphérique intérieure 24, illustrée à la figure 2, et une surface périphérique extérieure 25 pourvue de moyens de vissage, réalisés sous la forme d'un filetage 26, pour permettre le vissage du dispositif 2 dans l'os 21.

Le filetage 26 s'étend sensiblement à partir de l'extrémité proximale 18 jusqu'à une zone intermédiaire 27 de la surface périphérique extérieure 25 située à distance des extrémités proximale 18 et distale 19. Cette zone intermédiaire 27 est située à une distance de l'extrémité proximale 18 égale à une distance comprise entre 20 et 70% de la distance entre les extrémités proximale 18 et distale 19.

Le filetage 26 présente un pas de variable qui augmente en suivant le filetage 26 depuis l'extrémité proximale 18 vers la zone intermédiaire 27. La hauteur du filet du filetage 26 augmente également en suivant le filetage 26 depuis l'extrémité proximale 18 vers la zone intermédiaire 27.

La surface périphérique intérieure 24 délimite une portion de montage femelle 28 qui s'étend sensiblement à partir de l'extrémité proximale 18 et qui est dimensionnée pour coopérer par complémentarité de forme avec la portion de montage intermédiaire mâle 11 de l'organe d'accouplement 9. Dans une forme d'exécution non représentée sur les figures, la portion de montage femelle 28 est dimensionnée pour coopérer par complémentarité de forme avec la portion de montage mâle 7 de la cupule 3.

Le corps tubulaire 17 présente une surface distale 29 reliant surfaces périphériques intérieure 24 et extérieure 25. Cette surface distale 29 forme un chanfrein 30 qui permet de faciliter l'insertion dans l'os 21 par vissage du dispositif 2.

Une partie de la surface périphérique extérieure 25 qui s'étend de la zone intermédiaire 27 jusqu'à l'extrémité distale 19 est sensiblement lisse et présente quatre orifices 31 traversant le corps tubulaire 17.

Enfin, le dispositif 2 comporte 4 encoches 32 ménagées dans le filetage 26, au niveau de l'extrémité proximale 18. Ces encoches 32 sont conçues pour coopérer avec un outil de vissage et faciliter l'opération de vissage du dispositif 2 dans l'os 21.

Ainsi, un dispositif 2 selon l'invention décrit ci-dessus permet de fixer la cupule 3. Lorsque le corps tubulaire 17 du dispositif 2 est fixé dans l'os, l'ancrage correct du dispositif 2 est assuré grâce au filetage 26. La procédure de pose est simplifiée, puisque l'accouplement de la cupule 3 sur le dispositif 2 est simple.

En cas de besoin, le dispositif 2 peut être enlevé à l'aide d'une scie cloche de la manière décrite précédemment. Grace au dispositif 2, la partie de l'os 21 coopérant avec le filetage 26 et découpée peut être réduite puisque le filetage 26 ne s'étend pas sur la totalité de la longueur du dispositif 2. En cas de retrait du dispositif 2, le capital osseux est préservé et les risques de fragilisation de l'os 21 sont réduits.

Bien entendu, l'exemple de réalisation évoqué ci-dessus ne présente aucun caractère limitatif et d'autres détails et améliorations peuvent être apportés au dispositif 2 selon l'invention, sans pour autant sortir du cadre de l'invention où d'autres formes de dispositif 2 peuvent être réalisées.

## Revendications

1. Dispositif (2) pour la fixation d'une cupule (3) de prothèse d'épaule, le dispositif étant destiné à être implanté dans un humérus (21), notamment dans l'épiphyse (20) humérale, le dispositif comportant un corps tubulaire (17) s'étendant selon un axe longitudinal (A) entre une extrémité proximale (18), destinée à coopérer avec la cupule (3) ou un organe d'accouplement (9), et une extrémité distale (19) opposée destinée à être fixée dans l'os (21), le corps tubulaire (17) délimitant une surface périphérique intérieure (24) et une surface périphérique extérieure (25) pourvue de moyens de vissage pour permettre le vissage du dispositif (2) dans l'os (21), dans lequel la surface périphérique intérieure (24) délimite une portion de montage femelle (28) s'étendant sensiblement à partir de l'extrémité proximale (18) et conçue pour coopérer avec une portion de montage mâle (7) de la cupule (3) ou avec une portion de montage intermédiaire mâle (11) de l'organe d'accouplement (9), le dispositif (2) étant **caractérisé en ce que** les moyens de vissage comportent un filetage (26) s'étendant sensiblement à partir de l'extrémité proximale (18) jusqu'à une zone intermédiaire (27) de la surface périphérique extérieure (25) située à distance des extrémités proximale (18) et distale (19), **en ce que** le filetage (26) présente un pas de vis variable augmentant de l'extrémité proximale (18) vers la zone intermédiaire (27).

2. Dispositif (2) selon la revendication 1, **caractérisé en ce que** la portion de montage femelle (28) présente une forme tronconique femelle.

3. Dispositif (2) selon l'une des revendications 1 ou 2, **caractérisé en ce que** la hauteur du filet du filetage (26) augmente de l'extrémité proximale (18) vers la zone intermédiaire (27).

4. Dispositif (2) selon l'une des revendications 1 à 3, **caractérisé en ce que** la zone intermédiaire (27) est située à une distance de l'extrémité proximale (18) égale à une distance comprise entre 20 et 70% de la distance entre les extrémités proximale (18) et distale (19), de préférence comprise entre 40 et 50%.

5. Dispositif (2) selon l'une des revendications 1 à 4, **caractérisé en ce que** le corps tubulaire (17) présente une surface distale (29) reliant les surfaces périphériques intérieure (24) et extérieure (25) et formant un chanfrein (30).

6. Dispositif (2) selon l'une des revendications 1 à 5, **caractérisé en ce que** la partie de la surface périphérique extérieure (25) s'étendant de la zone intermédiaire (27) jusqu'à l'extrémité distale (19) est sensiblement lisse.

7. Dispositif (2) selon l'une des revendications 1 à 6, **caractérisé en ce que** la partie de la surface périphérique extérieure (25) s'étendant de la zone intermédiaire (27) jusqu'à l'extrémité distale (19) présente au moins un orifice (31) traversant le corps tubulaire (17).

8. Prothèse d'épaule (1) comprenant un dispositif (2) selon l'une des revendications 1 à 7 et une cupule (3) comportant une portion de montage mâle (7) conçue pour coopérer directement ou indirectement avec la portion de montage femelle (28) du dispositif (2).

9. Prothèse d'épaule (1) selon la revendication 8, comportant en outre un organe d'accouplement (9) destinée à être disposée entre le dispositif (2) et la cupule (3), l'organe d'accouplement (9) comportant une portion de montage intermédiaire mâle (11) conçue pour coopérer par complémentarité de forme avec la portion de montage femelle (28) du dispositif (2) et une portion de montage intermédiaire femelle (15) conçue pour coopérer par complémentarité de forme avec la portion de montage mâle (7) de la cupule.

## Patentansprüche

1. Vorrichtung (2) zur Fixierung einer Pfanne (3) einer Schulterprothese, wobei die Vorrichtung in einem Oberarmknochen (21), insbesondere in der Epiphyse (20) des Oberarmknochens, implantiert werden soll, wobei die Vorrichtung einen röhrenförmigen Körper (17) aufweist, der entlang einer Längsachse (A) zwischen einem proximalen Ende (18), das dazu bestimmt ist, mit der Pfanne (3) oder einem Verbindungsglied (9) zusammen zu wirken, und einem gegenüberliegenden distalen Ende (19), das dazu bestimmt ist, im Knochen (21) fixiert zu werden, verläuft, wobei der röhrenförmige Körper (17) eine Innenumfangsfläche (24) und eine Außenumfangsfläche (25) begrenzt, die mit Verschraubungsmitteln versehen ist, damit die Verschraubung der Vorrichtung (2) im Knochen (21) ermöglicht wird, wobei die Innenumfangsfläche (24) einen aufnehmenden Montageabschnitt (28) begrenzt, der im Wesentlichen von dem proximalen Ende (18) aus verläuft und so ausgelegt ist, dass er mit einem Montagesteckabschnitt (7) der Pfanne (3) oder mit einem Montagesteck-Zwischenabschnitt (11) des Verbindungsglieds (9) zusammenwirkt, wobei die Vorrichtung (2) **dadurch gekennzeichnet ist, dass** die Verschraubungsmittel ein Gewinde (26) aufweisen, das im Wesentlichen von dem proximalen Ende (18) bis zu einem Zwischenbereich (27) der Außenumfangsfläche (25) verläuft, der einen Abstand zum proximalen (18) und distalen Ende (19) aufweist, und dadurch, dass das Gewinde (26) eine veränderliche Steigung aufweist, die vom proximalen Ende (18) zum Zwischenbereich (27) hin größer wird.

2. Vorrichtung (2) nach Anspruch 1, **dadurch gekennzeichnet, dass** der aufnehmende Montageabschnitt (28) eine aufnehmende Kegelstumpfform aufweist.

3. Vorrichtung (2) nach Anspruch 1 oder 2, **dadurch gekennzeichnet, dass** die Gewindehöhe des Gewindes (26) vom proximalen Ende (18) zum Zwischenbereich (27) größer wird.

4. Vorrichtung (2) nach einem der Ansprüche 1 bis 3, **dadurch gekennzeichnet, dass** der Zwischenbereich (27) einen Abstand zum proximalen Ende (18) aufweist, der einem Abstand entspricht, der zwischen 20 und 70 %, vorzugsweise zwischen 40 und 50 %, des Abstands zwischen dem proximalen (18) und distalen Ende (19) beträgt.

5. Vorrichtung (2) nach einem der Ansprüche 1 bis 4, **dadurch gekennzeichnet, dass** der röhrenförmige Körper (17) eine distale Fläche (29) aufweist, die die Innenumfangsfläche (24) und Außenumfangsfläche (25) verbindet und eine Fase (30) bildet.

6. Vorrichtung (2) nach einem der Ansprüche 1 bis 5, **dadurch gekennzeichnet, dass** der Teil der Außenumfangsfläche (25), der von dem Zwischenbereich (27) zum distalen Ende (19) verläuft, im Wesentlichen glatt ist.

7. Vorrichtung (2) nach einem der Ansprüche 1 bis 6, **dadurch gekennzeichnet, dass** der Teil der Außenumfangsfläche (25), der von dem Zwischenbereich (27) zum distalen Ende (19) verläuft, mindestens eine Öffnung (31) aufweist, die durch den röhrenförmigen Körper (17) verläuft.

8. Schulterprothese (1), die eine Vorrichtung (2) nach einem der Ansprüche 1 bis 7 und eine Pfanne (3) umfasst, die einen Montagesteckabschnitt (7) aufweist, der so ausgelegt ist, dass er unmittelbar oder mittelbar mit dem aufnehmenden Montageabschnitt (28) der Vorrichtung (2) zusammenwirkt.

9. Schulterprothese (1) nach Anspruch 8, die ferner ein Verbindungsglied (9) aufweist, das dazu bestimmt ist, zwischen der Vorrichtung (2) und der Pfanne (3) angeordnet zu werden, wobei das Verbindungsglied (9) einen Montagesteck-Zwischenabschnitt (11) aufweist, der so ausgelegt ist, dass er formschlüssig mit dem aufnehmenden Montageabschnitt (28) der Vorrichtung (2) zusammenwirkt, und einen aufnehmenden Montagezwischenabschnitt (15), der so ausgelegt ist, dass er formschlüssig mit dem Montagesteckabschnitt (7) der Pfanne zusammenwirkt.

## Claims

1. Device (2) for fixing a shoulder prosthesis cup (3), the device being designed to be implanted in a humerus (21), in particular the humeral epiphysis (20), the device comprising a tubular body (17) extending along a longitudinal axis (A) between a proximal end (18) for cooperating with the cup (3) or a coupling member (9), and an opposite distal end (19) to be fixed in the bone (21), the tubular body (17) defining an inner peripheral surface (24) and an outer peripheral surface (25) provided with screw means to allow the device (2) to be screwed into the bone (21), wherein the inner peripheral surface (24) defines a female assembly portion (28) extending substantially from the proximal end (18) and designed to cooperate with a male assembly portion (7) of the cup (3) or with a male intermediate assembly portion (11) of the coupling member (9), the device (2) being **characterized in that** the screw means include a threading (26) extending substantially from the proximal end (18) to an intermediate region (27) of the outer peripheral surface (25) arranged at a distance from the proximal end (18) and the distal end (19), and **in that** the threading (26) has a variable screw pitch increasing from the proximal end (18) toward the intermediate region (27).

2. Device (2) according to claim 1, **characterized in that** the female assembly portion (28) has a female tapered shape.

3. Device (2) according to claim 1 or 2, **characterized in that** the height of the thread of the threading (26) increases from the proximal end (18) toward the intermediate region (27).

4. Device (2) according to one of claims 1-3, **characterized in that** the intermediate region (27) is situated at a distance from the proximal end (18) equal to a distance comprised between 20 and 70% of the distance between the proximal end (18) and the distal end (19), preferably comprised between 40 and 50%.

5. Device (2) according to one of claims 1-4, **characterized in that** the tubular body (17) has a distal surface (29) connecting the inner peripheral surface (24) and outer peripheral surface (25) and forming a bevel (30).

6. Device (2) according to one of claims 1-5, **characterized in that** the part of the outer peripheral surface (25) extending from the intermediate region (27) to the distal end (19) is substantially smooth.

7. Device (2) according to one of claims 1-6, **characterized in that** the part of the outer peripheral surface (25) extending from the intermediate region (27) to the distal end (19) has a least one orifice (31) passing through the tubular body (17).

8. Shoulder prosthesis (1) comprising a device (2) according to one of claims 1-7 and a cup (3) including a male assembly portion (7) designed to cooperate directly or indirectly with a female assembly portion (28) of the device (2).

9. Shoulder prosthesis (1) according to claim 8, also including a coupling member (9) designed to be arranged between the device (2) and the cup (3), the coupling member (9) including a male intermediate assembly portion (11) designed to cooperate by shape matching with the female assembly portion (28) of the device (2) and a female intermediate assembly portion (15) designed to cooperate by shape matching with the male assembly portion (7) of the cup.
